# EUROPÄISCHE PATENTSCHRIFT

(11) **EP 1 530 542 B1**
(45) Veröffentlichungstag und Bekanntmachung des Hinweises auf die Patenterteilung: **27.12.2017**
(21) Anmeldenummer: 03792287.9
(22) Anmeldetag: 09.08.2003
(51) Int. Cl.: B65B 55/10, B67B 1/03, A23L 3/06, A23L 3/08

(54) **STERILISIERVORRICHTUNG FÜR KAPPEN VON GETRÄNKEBEHÄLTERN**
STERILIZATION DEVICE FOR CAPS OF BEVERAGE CONTAINERS
DISPOSITIF DE STERILISATION POUR BOUCHONS DE CONTENANTS DE BOISSON

(30) Priorität: 19.08.2002 DE 10238633
(43) Veröffentlichungstag der Anmeldung: 18.05.2005
(73) Patentinhaber: KHS GmbH, 44143 Dortmund (DE)
(72) Erfinder: TOPF, Roland, 22177 Hamburg (DE)
(86) Internationale Anmeldenummer: PCT/EP2003/008862
(87) Internationale Veröffentlichungsnummer: WO 2004/018298

(56) Entgegenhaltungen:
- WO-A-98/46486
- US-A- 2 278 434
- US-A- 3 318 439
- PATENT ABSTRACTS OF JAPAN vol. 2000, no. 03, 30. März 2000 (2000-03-30) -& JP 11 342917 A (MITSUBISHI HEAVY IND LTD), 14. Dezember 1999 (1999-12-14)

## Beschreibung

Die Erfindung betrifft eine Sterilisiervorrichtung der im Oberbegriff des Anspruch 1 genannten Art.

Kappen von Getränkebehältern, wie z. B. Schraubkappen, Kronkorken, Dosendeckel und dergleichen müssen, wenn hochwertige Füllqualität gewünscht ist, vor der Anbringung auf dem Getränkebehälter sterilisiert werden. Die üblichen Sterilisierverfahren setzen dazu die Kappen einer Gasatmosphäre aus, die entweder selbst sterilisierend wirkt oder auf die Oberflächen der Kappen aufgebrachte sterilisierende Flüssigkeiten wie z.B. H₂O₂ abtrocknet. Die Kappen müssen daher für eine längere Zeit einer bestimmten Atmosphäre ausgesetzt sein.

Da bei modernen Behälterbehandlungsmaschinen die Kappen in Spuren, zumeist einspurig, geführt werden, um einen geordneten Ablauf zu ermöglichen, sollten sie auch in der Sterilisiervorrichtung in Spuren geführt werden. Berücksichtigt man die heutigen Verarbeitungsgeschwindigkeiten von Kappenverschließmaschinen, so kommt man bei einspuriger Führung angesichts der erforderlichen Verweilzeit der Kappen in der Sterilisiervorrichtung auf Bahnlängen von mehreren Metern. Ist die Bahn als Rutsche oder angetriebener Transporteur ausgebildet, so ergeben sich erhebliche Platz- und Konstruktionsprobleme.

Eine Sterilisiervorrichtung für Kappen ist aus der WO 00/46142 A2 bekannt.
In einem geschlossenen von der Behandlungsatmosphäre durchströmten Gehäuse ist die Bahn mehrfach um eine lotrechte Achse umlaufend angeordnet. Dies gibt den Vorteil, eine große Bahnlänge auf kleinem Raum unterzubringen. Die Platzprobleme können auf diese Weise gelöst werden.
Nachteilig bei dieser Konstruktion ist die schraubenförmige stationäre Ausbildung der Bahn, auf der sich die Kappen rutschend über die gesamte Bahnlänge bewegen. Angesichts der zur Verringerung des Raumbedarfes erforderlichen flachen Neigung der Schraube, rutschen die Kappen nicht allein aufgrund des Schrägwinkels der Bahn, sondern erfordern einen Zusatzantrieb, den die zitierte Schrift in unterschiedlichen Ausführungen zeigt, entweder mit Luftstrahldüsen oder mit einer umlaufenden, in Schlitzeingriff die Kappen mitnehmenden Trommel. Es ergibt sich insgesamt eine außerordentlich komplizierte Konstruktion, die außerdem anfällig gegen Verklemmen und Staugefahren ist. Aus der US-A-2278434 ist eine Vorrichtung zur thermischen Behandlung abgefüllter Dosen bekannt, die ein geschlossenes, gasdurchströmtes Gehäuse, welches von einer Bahn durchlaufen ist zeigt. Die Vorrichtung weist ferner stationäre Überführungsrutschen zur Übernahme der Dosen von einem oberen Tragring und zur Übergabe an den darunterliegenden Tragring auf. Die Aufgabe der vorliegenden Erfindung besteht darin, eine gattungsgemäße Sterilisiervorrichtung zu schaffen, die bei einfacherem Aufbau weniger stör- und stauanfällig ist.
Diese Aufgabe wird mit den Merkmalen des Anspruches 1 gelöst.
Erfindungsgemäß laufen die Kappen nacheinander auf mehreren übereinander angeordneten, waagerecht umlaufenden Tragringen, die selbst angetrieben sind und somit die Kappen liegend über einen größeren Umlaufwinkel transportieren. In einem Sektorbereich der Vorrichtung sind übereinander Überführungsrutschen vorgesehen, die jeweils von einem Tragring die Kappen übernehmen und auf kurzem Wege abwertsrutschend auf den nächst tieferen Tragring übergeben. Diese Konstruktion ist im Aufbau sehr einfach. Sie erfordert im wesentlichen mehrere übereinander synchron laufend angetriebene Tragringe sowie die stationären Überführungsrutschen. Auch die Stör- und Staugefahr ist wesentlich niedriger als bei der bekannten Konstruktion, da bei dem liegenden Transport auf den Tragringen keine Störungsgefahr besteht und in den relativ kurzen Überführungsrutschen die Störgefahren sehr niedrig sind. Die Tragringe können im wesentlichen im Abstand der Kappenhöhen übereinander angeordnet sein, so daß in einem relativ kleinen Gehäuse eine sehr lange Bahn untergebracht werden kann.

Die Tragringe müssen lediglich dazu geeignet sein, die Kappen sicher zu tragen, ohne daß sie nach unten durchfallen können. Die Seitenführungen zur Begrenzung der Bahn auf den Tragringen können als einfache Bleche ausgebildet sein. Vorteilhaft sind dabei die Merkmale des Anspruches 2 vorgesehen, wonach die Tragringe und/oder die Seitenführungen gasdurchlässig ausgebildet sind. Damit wird während des Transportes der Kappen eine gute Umströmung mit der durch das umschließende Gehäuse strömenden Atmosphäre gewährleistet. Dabei können beispielsweise vorteilhaft die Tragringen als ringförmig ausgebildete parallele Stangen ausgebildet sein. Die zur seitlichen Begrenzung angeordneten Bleche können gelocht oder beispielsweise als Gitter ausbildet sein.

Die Zuführung von Kappen zum obersten Tragring bzw. die Abführung vom untersten Tragring kann mit allen für diese Zwecke geeigneten Mitteln wie z.B. mit Überführungssternen erfolgen. Vorteilhaft sind jedoch die Merkmale der Ansprüche 3 bzw. 4 vorgesehen, die eine besonders einfache Konstruktion ergeben mit guter Aufgabe der Kappen auf bzw. Abnahme von den jeweiligen Tragringen.

Vorteilhaft sind die Merkmale des Anspruches 5 vorgesehen. Dadurch vereinfacht sich die Konstruktion gegenüber im Rahmen der vorliegenden Erfindung ebenfalls möglichen Sonderkonstruktionen mit individuellem Antrieb der Tragringe, die z.B. an einzeln angetriebenen Rädern ausgebildet sein können.

Vorteilhaft sind dabei die Merkmale des Anspruches 6 vorgesehen, danach sind die Tragringe auf dem Außenumfang einer umlaufenden Trommel übereinander befestigt, was eine sehr einfache Konstruktion ergibt. Die Trommel kann dabei die innere Seitenführung ausbilden, die mit den Tragringen mitläuft und somit die Staugefahr weiter verringert. Die Trommel bietet auch den Vorteil, dass sie als geschlossene Trommel ausgebildet sein kann und somit in dem geschlossenen Gehäuse den von der Behandlungsatmosphäre gefüllten Raum nach innen begrenzt.

In der Zeichnung ist die Erfindung beispielsweise und schematisch dargestellt. Es zeigen:
- Fig. 1: Einen Achsschnitt durch eine erfindungsgemäße Sterilisiervorrichtung gemäß Linie 1-1 in Figuren 2 und 3,
- Fig. 2: eine Achsansicht der inneren Teile der Sterilisiervorrichtung, ohne Gehäuse dargestellt, gemäß Schnitt nach Linie 2-2 in Fig. 1 und
- Fig. 3: eine Seitenansicht gemäß Pfeil 3 in Fig. 2, dargestellt mit Gehäuse.

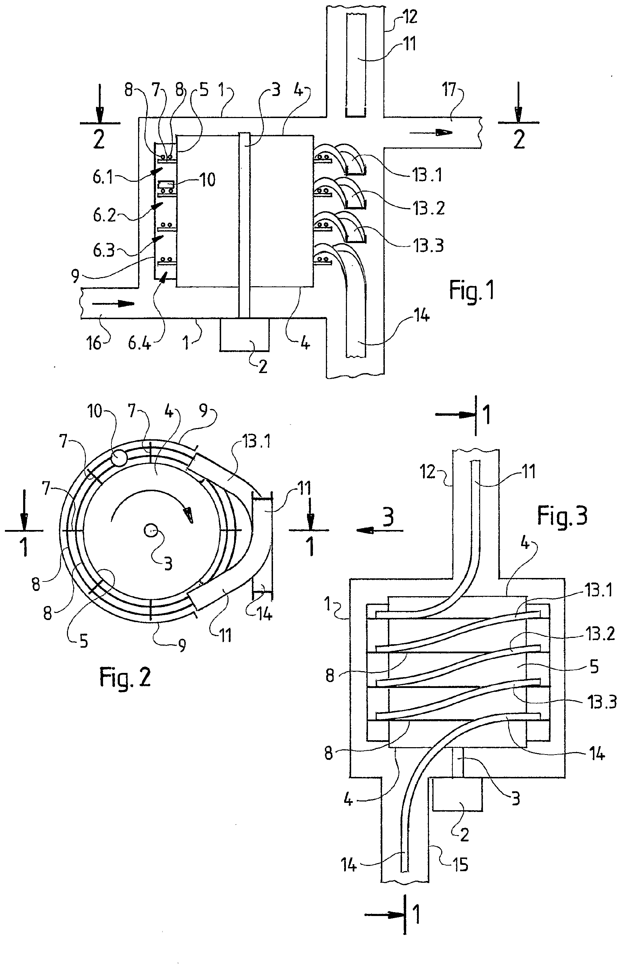

Wie aus den Figuren, die dieselbe Ausführungsform in unterschiedlichen Ansichten zeigen, ersichtlich, ist in einem Gehäuse 1 eine lotrecht stehende, von einem Motor 2 angetriebene Welle 3 angeordnet. Diese treibt eine Trommel bestehend aus Endwänden 4 und einer zylindrischen Umfangswand 5 an. Die Trommel 4, 5 dreht in Richtung des Pfeiles in Fig. 2.

Auf dem Umfang der Trommel 4,5 sind vier Tragringe 6.1-6.4 befestigt. Im Ausführungsbeispiel bestehen diese jeweils aus mehreren auf der Umfangswand 5 der Trommel radial erstreckt befestigten Stiften 7 und zwei auf diesen befestigten, konzentrisch um die Umfangswand 5 der Trommel umlaufenden Ringstäben 8.

Über mehr als die Hälfte des Umfanges umlaufend um die Trangringe 6.1-6.4 ist ein äußeres Führungsblech 9 stationär angeordnet. In Fig. 1 und Fig. 2 sind jeweils eine Kappe 10, auf den Ringstäben 8 eines Tragringes 6 liegend dargestellt. Man erkennt, dass die Kappen 10 sicher auf den Ringstäben 8 liegen und von dem Führungsblech 9 als äußerer Seitenführung und der Umfangswand 5 als innerer Seitenführung auf der so gebildeten konzentrischen Bahn geführt werden. Der Transport der Kappen 10 erfolgt durch die Umlaufbewegung der Tragringe 6 mit der Trommel 4,5.

Eine Zuführung 11 in Form einer Rutsche mit für diese Zwecke üblichem rechteckförmigen Führungsprofil kommt, wie die Figuren 1 und 3 zeigen, von oben durch einen z. B. mit nicht dargestellten Einführungsschleusen versehenen Kanal 12 in das Innere des Gehäuses 1 und verläuft dort, wie die Figuren 2 und 3 zeigen abgebogen bis auf den obersten Tragring 6.1, um dort die unter Schwerkraft nacheinander nachrutschenden Kappen 10 auf den obersten Tragring 6.1 zuzuführen.

Nach dem Ende der Zuführung 11 liegen die Kappen auf dem obersten Tragring 6.1 und laufen mit diesem um, bis sie an den Anfang einer ersten Überführungsrutsche 13.1 gelangen, die ebenfalls als für solche Rutschenzwecke üblicherweise geeignetes Rechteckprofil ausgebildet ist. Die Überführungsrutsche 13.1 verläuft von ihrem Anfang in der aus den Figuren 1, 2 und 3 ersichtlichen, nach außen unten verschwenkten Weise von einer Abnahmeposition auf dem obersten Tragring 6.1 zu einer Aufgabeposition auf den darunterliegenden Tragring 6.2, die um einen Winkelsektor später liegt.

Unterhalb der obersten Überführungsrutsche 13.1 sind zwei weitere Überführungsrutschen 13.2 und 13.3 in identischer Ausführung vorgesehen, die jeweils von einem oberen Tragring übernehmen und auf den darunter liegenden Tragring übergeben. Auf diese Weise gelangen die Kappen vom obersten Tragring 6.1 auf den darunterliegenden Tragring 6.2, sodann zu 6.3 und schließlich zu 6.4 und werden von dort mit einer im Anfangsbereich ähnlich einer Überführungsrutsche ausgebildeten Abführung 14 vom untersten Tragring 6.4 abgenommen und nach unten durch einen Kanal 15 aus dem Gehäuse 1 abgeführt.

Wie insbesondere aus Fig. 2 ersichtlich, liegen die Überführungsrutschen 13.1 bis 13.3 in dem Sektorbereich der Trommel 4,5, der nicht von dem Führungsblech 9 umlaufen ist.

Aus Fig. 1 ist ersichtlich, daß der Innenraum des Gehäuses 1 durch einen Zustromkanal 16 mit Gas versorgt wird, welches durch einen Abstromkanal 17 aus dem Gehäuse 1 abströmt. Die Gasführung ist, wie Fig. 1 zeigt, im wesentlichen im Gegenstrom zum Lauf der Kappen 10 durch das Gehäuse 1 angeordnet. Als durchströmendes Gas kann z. B. ein sterilisierendes Gas verwendet werden. Die Kappen 10 können jedoch auch vor Einlauf in das Gehäuse 1 z. B. mit einer sterilisierenden Flüssigkeit besprüht sein, die im Inneren des Gehäuses 1 z. B. mit durchströmender Heißluft abgetrocknet werden soll.

Es können nicht dargestellte Leiteinrichtungen für den Gasstrom im Inneren des Gehäuses 1 vorgesehen sein, die den Gasstrom auf bestimmte vorteilhafte Bahnen lenken, beispielsweise im wesentlichen in dem Ringraum zwischen der Umfangswand 5, der Trommel 4,5 und dem umgebenden Führungsblech 9 aufwärts durch die Tragringe 6.4-6.1 strömend. Das Führungsblech 9 kann beispielsweise auch gelocht oder als Gitter ausgebildet sein, um eine freie Gasdurchströmung zu ermöglichen.

In der in den Figuren dargestellten Ausführungsform werden die Kappen 10 auf den dargestellten Rutschen 11, 14, 13.1-13.3 sowie auf den Tragringen 6.1-6.4 einspurig geführt. Bei breiterer Ausbildung dieser Elemente können die Kappen auch mehrspurig geführt werden.

In der dargestellten Ausführungsform sind die Rutschen 11, 13.1-13.3 und 14 als rechteckige Blechprofile dargestellt. Diese Rutschen können jedoch auch in üblicher Ausführung als Stangenführungen ausgebildet sein mit Tragstangen und seitlichen Begrenzungsstangen. Dadurch wird die Gasumströmung der Kappen 10 auch in diesem Bereich verbessert. Bei den Überführungsrutschen 13.1-13.3 können in diesem Fall die seitlichen Begrenzungen als von oben nach unten durchgehende Bleche ausgebildet sein, die in diesem Bereich ähnlich wie das Führungsblech 9 vorgesehen sind.

## Patentansprüche

1. Sterilisiervorrichtung für auf einer Bahn in wenigstens einer Spur geführte Kappen (10) für Getränkebehälter, mit einem geschlossenen gasdurchströmten Gehäuse (1), das von der Bahn (11, 6.1-6.4, 13.1-13.3, 14) durchlaufen ist, wobei die Bahn innerhalb des Gehäuses (1) abwärts mehrfach um eine lotrechte Achse (3) umlaufend angeordnet ist, wobei pro Umlauf ein umlaufend angetriebener Tragring (6.1-6.4) zwischen radial innen sowie außen angeordneten, die Bahn (6.1-6.4) begrenzenden Seitenführungen (5, 9) angeordnet ist
und daß an übereinanderliegenden Sektorausschnitten der äußeren Seitenführung (9) stationäre Überführungsrutschen (13.1-13.3) angeordnet sind, die die Kappen (10) jeweils von einem oberen Tragring (6.1) übernehmen und in nach außen sowie nach unten verschwenkter Anordnung auf den darunterliegenden Tragring (6.2) übergeben, wobei eine Zuführung (11) Kappen (10) dem obersten Tragring (6.1) zuführt und eine Abführung (14) Kappen (10) aus dem untersten Tragring (6.4) abführt, wobei die Tragringe (6.1-6.4) synchron zueinander angetrieben sind, und wobei die Tragringe (6.1-6.4) an einer drehangetriebenen, die innere Seitenführung (5) bildenden Trommel (4, 5) befestigt sind.

2. Sterilisiervorrichtung nach Anspruch 1, **dadurch gekennzeichnet, daß** die Tragringe (6.1-6.4) und/oder die Seitenführungen (5, 9) und/oder die Überführungsrutschen (13.1-13.3) und/oder die Zuführung (11) und/oder die Abführung (13) gasdurchlässig ausgebildet sind.

3. Sterilisiervorrichtung nach Anspruch 1, **dadurch gekennzeichnet, daß** die Zuführung (11) in ihrem Endstück entsprechend dem Endstück einer Überführungsrutsche (13) ausgebildet ist.

4. Sterilisiervorrichtung nach Anspruch 1, **dadurch gekennzeichnet, daß** die Abführung (14) in ihrem Anfangsstück entsprechend dem Anfangsstück einer Überführungsrutsche (13) ausgebildet ist.

## Claims

1. Sterilisation device for beverage container caps (10) which are conveyed on at least track on a web, said device comprising a closed housing (1) traversed by a gas, wherein the web (11, 6.1-6.4, 13.1-13.3, 14) runs through said housing and is arranged inside the housing (1) in such a way that it circulates downstream several times about a perpendicular axis (3), wherein, per circulation, a peripherally driven carrier ring (6.1-6.4) is arranged between lateral guides (5, 9) which define said web (6.1-6.4),
and that stationary transfer chutes (13.1-13.3) are arranged on superimposed sections of the outer lateral guide (9), said chutes collecting the caps (10) in each case from a top supporting ring (6.1) and delivering said caps in an array pivoting outward and downward onto the supporting ring (6.2) lying below, wherein a feed device (11) feeds the caps (10) to the top supporting ring (6.1) and a removal device (14) removes the caps (10) from the bottom supporting ring (6.4), wherein the supporting rings (6.1-6.4) are driven synchronously in relation to one another, and wherein the supporting rings (6.1-6.4) are secured to a rotationally driven drum (4, 5) forming the inner lateral guide (5).

2. Sterilisation device according to claim 1, **characterised in that** the supporting rings (6.1-6.4) and/or the lateral guides (5, 9) and/or the transfer chutes (13.1-13.3) and/or the feed device (11) and/or the removal device (14) are configured as gas-permeable.

3. Sterilisation device according to claim 1, **characterised in that** the feed device (11) is configured in its end piece so as to correspond to the end piece of a transfer chute (13).

4. Sterilisation device according to claim 1, **characterised in that** the removal device (14) is configured in its start piece so as to correspond to the start piece of a transfer chute (13).

## Revendications

1. Dispositif de stérilisation pour des bouchons (10) guidés sur une bande dans au moins une voie pour des contenants de boisson, avec un boîtier (1) fermé traversé par du gaz qui est traversé par la bande (11, 6.1-6.4, 13.1-13.3, 14), dans lequel la bande dans le boîtier (1) est disposée de manière tournante autour d'un axe vertical (3) vers le bas de façon multiple, dans lequel par rotation un anneau porteur (6.1-6.4) entraîné en rotation est disposé entre des guidages latéraux (5, 9) délimitant la bande (6.1-6.4), disposés radialement à l'intérieur ainsi qu'à l'extérieur,
et qu'au niveau de sections de secteur superposées du guidage latéral extérieur (9), des glissières de transport (13.1-13.3) stationnaires sont disposées, lesquelles prennent les bouchons (10) respectivement d'un anneau porteur (6.1) supérieur et les remettent dans un agencement pivoté vers l'extérieur ainsi que vers le bas à l'anneau porteur (6.2) se trouvant dessous, dans lequel une alimentation (11) fournit des bouchons (10) à l'anneau porteur (6.1) supérieur et une évacuation (14) évacue des bouchons (10) de l'anneau porteur (6.4) inférieur, dans lequel
les anneaux porteurs (6.1-6.4) sont entraînés de manière synchrone l'un à l'autre, et dans lequel les anneaux porteurs (6.1-6.4) sont fixés à un tambour (4, 5) entraîné en rotation formant le guidage latéral (5) intérieur.

2. Dispositif de stérilisation selon la revendication 1, **caractérisé en ce que** les anneaux porteurs (6.1-6.4) et/ou les guidages latéraux (5, 9) et/ou les glissières de transport (13.1-13.3) et/ou l'alimentation (11) et/ou l'évacuation (13) sont réalisés de manière perméable au gaz.

3. Dispositif de stérilisation selon la revendication 1, **caractérisé en ce que** l'alimentation (11) est réalisée dans sa partie terminale de manière à correspondre à la partie terminale d'une glissière de transport (13).

4. Dispositif de stérilisation selon la revendication 1, **caractérisé en ce que** l'évacuation (14) est réalisée dans sa partie initiale de manière à correspondre à la partie initiale d'une glissière de transport (13).
